# EUROPEAN PATENT APPLICATION

(11) **EP 3 848 015 A1**
(43) Date of publication of application: **14.07.2021**
(21) Application number: 21159525.1
(22) Date of filing: 18.12.2017
(51) Int. Cl.: A61K 8/26, A61K 8/34, A61K 8/39, A61Q 15/00, A61K 8/86

(54) **ANTIPERSPIRANT / DEODORANT COMPOSITION**

(30) Priority: 27.12.2016 US 201662439226 P
(62) Divisional of application: 17826401.6
(71) Applicant: Colgate-Palmolive Company, New York, NY 10022 (US)
(72) Inventor: GARCIA, Jesus, Miguel Hidalgo DF (MX); KENNEDY, Sharon, Randallstown, 21133 (US); DENNIS, Mavis, Sayreville, 08872 (US); HOLERCA, Marian, Somerset, 08873 (US)
(74) Representative: Klaus, Stephan

(57) **Abstract**

An antiperspirant and/or deodorant composition, including a carrier, an antiperspirant and/or deodorant active, one or two surfactants, and a preservative. The composition may optionally include a fragrance and/or an emollient, and may have a pearlescent appearance.

## Description

### BACKGROUND

Antiperspirant or deodorant compositions may be used to reduce body odor. Antiperspirant or deodorant compositions may be applied to axillary (underarm) regions to limit perspiration, limit or kill odor causing bacteria in the region, or apply a fragrance. Antiperspirant or deodorant compositions may be delivered topically as roll-on, gel, or aerosol formulations.

In order to be effective, conventional antiperspirant or deodorant compositions include a large number of ingredients working together to create a stable composition with the desired functional characteristics. However, the complexity of such compositions require extensive amounts of time and logistical efforts to assure the effectiveness and compatibility of the various ingredients when used together.

Accordingly, there is a desire for antiperspirant and/or deodorant compositions with a minimum number of ingredients that still maintains desire levels of stability, efficiency, and aesthetic characteristics

### BRIEF SUMMARY

This section is intended merely to introduce a simplified summary of some aspects of one or more embodiments of the present disclosure. Further areas of applicability of the present invention will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing an antiperspirant and/or deodorant composition, including between 60 weight % and 95 weight % carrier, based on a total weight of the antiperspirant and/or deodorant composition; between 2 weight % and 25 weight % antiperspirant and/or deodorant active, based on the total weight of the antiperspirant and/or deodorant composition; between 1 weight % and 7 weight % surfactant, based on the total weight of the antiperspirant and/or deodorant composition; and between 0.1 weight % and 0.5 weight % preservative, based on the total weight of the antiperspirant and/or deodorant composition.

The antiperspirant and/or deodorant composition may not include rheology modifiers, viscosity modifiers, or thickening agents.

The antiperspirant and/or deodorant composition may not include gelling agents.

A viscosity of the antiperspirant and/or deodorant composition may be from about 900 to about 4000cps.

The antiperspirant and/or deodorant composition may exhibit pearlescense.

The carrier may consist essentially of water.

The antiperspirant and/or deodorant active may consist essentially of an antiperspirant active.

The antiperspirant active may consist essentially of aluminum chlorohydrate.

The antiperspirant and/or deodorant active may consist essentially of a deodorant active.

The deodorant active may consist essentially of potassium aluminum sulfate.

The surfactant may include one or more low HLB surfactants and one or more high HLB surfactants, and the one or more low HLB surfactants may have an HLB value from about 3 to about 6 and the one or more high HLB surfactants may have an HLB value from about 12 to about 16.

The surfactant may consist essentially of Steareth-2 and Steareth-21.

The preservative may consist essentially of caprylyl glycol.

The antiperspirant and/or deodorant composition may further include at least one of a fragrance, and an emollient.

The antiperspirant and/or deodorant composition may consist essentially of between 60 weight % and 90 weight % water as the carrier; between 8 weight % and 12 weight % aluminum chlorohydrate as the deodorant and/or antiperspirant active; between 1 weight % and 6 weight % Steareth-2 and Steareth-21 as the surfactant; between 0.1 weight % and 0.5 weight % caprylyl glycol as the preservative; and between 0 weight % and 30 weight % emollient.

The antiperspirant and/or deodorant composition may consist essentially of between 60 weight % and 95 weight % water, between 2 weight % and 5 weight % potassium aluminum sulfate; between 1 weight % and 6 weight % Steareth-2 and Steareth-21; an effective amount of preservative; and between 0 weight % and 15 weight % emollient.

The foregoing and/or other aspects and utilities embodied in the present disclosure may be achieved by providing an antiperspirant and/or deodorant composition, including between 60 weight % and 95 weight % carrier, based on a total weight of the antiperspirant and/or deodorant composition; between 2 weight % and 25 weight % antiperspirant and/or deodorant active, based on the total weight of the antiperspirant and/or deodorant composition; between 1 weight % and 6 weight % surfactant, based on the total weight of the antiperspirant and/or deodorant composition; an effective amount of a preservative; and between 0 weight % and 15 weight % emollient, based on the total weight of the antiperspirant and/or deodorant composition, wherein the antiperspirant and/or deodorant composition does not comprise rheology modifiers, viscosity modifiers, thickening agents, or gelling agents, wherein a viscosity of the antiperspirant and/or deodorant composition is from about 900 to about 4000cps, and wherein the antiperspirant and/or deodorant composition exhibits pearlescense.

The carrier may consist essentially of water, the antiperspirant and/or deodorant active may consist essentially of at least one of aluminum chlorohydrate and potassium aluminum sulfate, the surfactant comprises one or more low HLB surfactants and one or more high HLB surfactants, the one or more low HLB surfactants have an HLB value from about 3 to about 6 and the one or more high HLB surfactants have an HLB value from about 12 to about 16, and the preservative may consist essentially of caprylyl glycol.

The foregoing and/or other aspects and utilities embodied in the present disclosure may be achieved by providing a pearlescent antiperspirant and/or deodorant composition, including between 2 weight % and 25 weight % antiperspirant and/or deodorant active, based on the total weight of the antiperspirant and/or deodorant composition; and between 2.5 weight % and 3.5 weight % surfactant, based on the total weight of the antiperspirant and/or deodorant composition, wherein the surfactant includes one or more low HLB surfactants and one or more high HLB surfactants, and wherein the one or more low HLB surfactants have an HLB value from about 3 to about 6 and the one or more high HLB surfactants have an HLB value from about 12 to about 16, wherein the antiperspirant and/or deodorant composition does not comprise rheology modifiers, viscosity modifiers, thickening agents, or gelling agents, wherein a viscosity of the antiperspirant and/or deodorant composition is from about 900 to about 4000cps.

The antiperspirant and/or deodorant active may consist essentially of at least one of aluminum chlorohydrate and potassium aluminum sulfate, and the surfactant includes Steareth-2 and Steareth-21.

### DETAILED DESCRIPTION

Reference will now be made in detail to the various embodiments in the present disclosure, examples of which may be illustrated in any accompanying drawings and figures. The embodiments are described below to provide a more complete understanding of the components, processes, compositions, and apparatuses disclosed herein. Any examples given are intended to be illustrative, and not restrictive. However, it will be apparent to one of ordinary skill in the art that the invention may be practiced without these specific details. In other instances, well-known methods, procedures, and components have not been described in detail so as not to unnecessarily obscure aspects of the embodiments.

Throughout the specification and claims, the following terms take the meanings explicitly associated herein, unless the context clearly dictates otherwise. The phrases "in some embodiments" and "in an embodiment" as used herein do not necessarily refer to the same embodiment(s), though they may. Furthermore, the phrases "in another embodiment" and "in some other embodiments" as used herein do not necessarily refer to a different embodiment, although they may. As described below, various embodiments may be readily combined, without departing from the scope or spirit of the present disclosure.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes embodiments containing A, B, or C, multiple examples of A, B, or C, or combinations of A/B, A/C, B/C, A/B/B/ B/B/C, A/B/C, etc. In addition, throughout the specification, the meaning of "a," "an," and "the" include plural references. The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, component, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if' may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5-6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

Unless otherwise specified, all percentages and amounts expressed herein and elsewhere in the specification should be understood to refer to percentages by weight. The amounts given are based on the active weight of the material.

With regard to procedures, methods, techniques, and workflows that are in accordance with some embodiments, some operations in the procedures, methods, techniques, and workflows disclosed herein may be combined and/or the order of some operations may be changed.

The present disclosure is directed at an antiperspirant and/or deodorant composition. The antiperspirant and/or deodorant composition may include one or more active antiperspirant ingredients, one or more active deodorant ingredients, or a mixture of active antiperspirant and/or deodorant ingredients.

According to embodiments of the disclosure, the antiperspirant and/or deodorant composition may include five types of ingredients: one or more antiperspirant and/or deodorant actives, one or more carriers, one or more surfactants, and one or more preservatives. In other embodiments, the antiperspirant and/or deodorant composition may also include optional ingredients, such as one or more fragrances or one or more emollients. In some embodiments, the antiperspirant and/or deodorant composition is limited to one or more antiperspirant and/or deodorant actives, one or more carriers, one or more surfactants, and one or more preservatives. In some embodiments, the antiperspirant and/or deodorant has only five types of ingredients. In one such embodiment, only a deodorant active is present and there are a total of five types of ingredients. In another such embodiment, only an antiperspirant active is present and there are a total of five types of ingredients.

In other embodiments, the antiperspirant and/or deodorant composition is limited to one or more antiperspirant and/or deodorant actives, one or more carriers, one or more surfactants, and one or more preservatives and at least one of one or more fragrances and one or more emollients.

In some embodiments, the antiperspirant and/or deodorant composition comprises an antiperspirant active and/or a deodorant active, two surfactants, a carrier, and a preservative. In other embodiments, the antiperspirant and/or deodorant composition comprises an antiperspirant active and/or a deodorant active, two surfactants, a carrier, a preservative, a fragrance and/or an emollient.

The antiperspirant and/or deodorant composition may be provided as a liquid or a gel. In one embodiment, the antiperspirant and/or deodorant composition is a liquid roll-on formulation. The liquid roll-on formulation may be contained in any roll-on dispenser that has a ball for applying the antiperspirant and/or deodorant composition to the surface of the skin.

The antiperspirant and/or deodorant composition may include one or more carriers or solvents. For example, the antiperspirant and/or deodorant composition may be provided as an oil-in-water emulsion using primarily water as the carrier. In other embodiments, the carrier may include other co-solvents which are miscible with water. However, in various preferred embodiments, the carrier consists only of water or consists essentially of water, such as a carrier that consists of at least 99% water.

In one embodiment, the amount of carrier in the antiperspirant and/or deodorant composition is the amount to make a 100% by weight composition after all of the ingredients, including any optional ingredients, are added to the composition. For example, the antiperspirant and/or deodorant composition may include at least 60 weight % water, from about 60 weight % to about 90 weight % water, from about 70 weight % to about 80 weight % water, or about 75 weight % water.

In one embodiment, the antiperspirant and/or deodorant composition includes one or more antiperspirant actives. In some embodiments, the antiperspirant actives are compatible with the other ingredients in the antiperspirant and/or deodorant composition. For example, in some embodiments, the antiperspirant actives are selected to maintain the overall pH of the antiperspirant and/or deodorant composition in a range from about 3 to about 5.

In one embodiment, the antiperspirant actives includes at least one of aluminum chlorohydrate, aluminium chloride, and aluminum zirconium. In other embodiments, the antiperspirant actives consist essentially of aluminum chlorohydrate, with only trace amounts of other materials.

In some embodiments, the antiperspirant actives are the only active odor control ingredient. For example, in one embodiment, aluminum chlorohydrate is the only active odor control ingredient in the antiperspirant and/or deodorant composition. In other embodiments, the aluminum chlorohydrate is the only active odor control ingredient in the antiperspirant and/or deodorant composition except for the preservative.

In one embodiment, the antiperspirant and/or deodorant composition includes an effective amount of antiperspirant actives. For example, the antiperspirant and/or deodorant composition may include an amount of antiperspirant actives effective to reduce the flow of perspiration in the axillary region. In other embodiments, the antiperspirant and/or deodorant composition may include an amount of antiperspirant actives effective to reduce malodor or to act as an antibacterial. For example, a Wilcoxon Signed Rank test may be used to determine an effective amount of antiperspirant actives. In one embodiment, a level of significance was established at Z < 70% (for 30% sweat reduction), Z < 80% (for 20% sweat reduction), and Z < 100% (for general efficacy) to establish an effective amount of antiperspirant actives in the antiperspirant and/or deodorant composition.

In other embodiments, the antiperspirant and/or deodorant composition includes from about 2% to about 25% antiperspirant actives, based on a total weight of the antiperspirant and/or deodorant composition. For example, the antiperspirant and/or deodorant composition may include from about 10 weight % to about 20 weight % antiperspirant actives or from about 8 weight % to about 16 weight % antiperspirant actives. In one embodiment, the antiperspirant and/or deodorant composition includes about 12 weight % antiperspirant actives. For example, the antiperspirant and/or deodorant composition may include from about 8 weight % to about 25 weight % aluminum chlorohydrate, from about 10 weight % to about 16 weight % aluminum chlorohydrate, or about 12 weight % aluminum chlorohydrate.

In one embodiment, the antiperspirant and/or deodorant composition includes one or more deodorant actives. In various embodiments, the deodorant actives are compatible with the other ingredients in the antiperspirant and/or deodorant composition. For example, in some embodiments, the deodorant actives are selected to maintain the overall pH of the antiperspirant and/or deodorant composition in a range from about 3 to about 5. In other embodiments, the deodorant active is selected to be dissolvable in the carrier.

In one embodiment, the deodorant actives includes at least one of alum, potassium aluminum sulfate, zinc oxide, ethylhexylglycerin, octenidine hydrochloride, zinc citrate, zinc pyrithione, and silver compounds. For example, in some embodiments, the deodorant actives include potassium aluminum sulfate or potassium aluminum sulfate crystals.

In other embodiments, the deodorant actives consist essentially of only one of at least one of alum, potassium aluminum sulfate, zinc oxide, ethylhexylglycerin, octenidine hydrochloride, zinc citrate, zinc pyrithione, and silver compounds, with only trace amounts of other materials. In some embodiments, the deodorant actives are the only active odor control ingredient. For example, in one embodiment, potassium aluminum sulfate is the only active odor control ingredient in the antiperspirant and/or deodorant composition. In other embodiments, the potassium aluminum sulfate is the only active odor control ingredient in the antiperspirant and/or deodorant composition except for the preservative which may have some secondary odor control properties.

In various embodiments, the deodorant actives are fully dissolved in the carrier.

In one embodiment, the antiperspirant and/or deodorant composition includes an effective amount of deodorant actives. For example, the antiperspirant and/or deodorant composition may include an amount of deodorant actives effective to reduce malodor or to act as an antibacterial in the axillary region. A Wilcoxon Signed Rank test may be used to determine an effective amount of deodorant actives.

In other embodiments, the antiperspirant and/or deodorant composition includes from about 2 % to about 25 % deodorant actives, based on a total weight of the antiperspirant and/or deodorant composition. For example, the antiperspirant and/or deodorant composition may include from about 10 weight % to about 20 weight % deodorant actives or from about 8 weight % to about 16 weight % deodorant actives. In one embodiment, the antiperspirant and/or deodorant composition includes about 12 weight % deodorant actives. For example, the antiperspirant and/or deodorant composition may include from about 8 weight % to about 25 weight % potassium aluminum sulfate, from about 10 weight % to about 16 weight % potassium aluminum sulfate, or about 12 weight % potassium aluminum sulfate.

In one embodiment, the antiperspirant and/or deodorant composition includes one or more surfactants. In some embodiments, the surfactants are selected to provide adequate viscosity to the antiperspirant and/or deodorant composition. In other embodiments, the surfactants are selected to enable the solubilization of other ingredients in the antiperspirant and/or deodorant composition. In yet other embodiments, the surfactants are selected to resist degradation in a low pH environment.

For example, the inventors have discovered that a system of surfactants with high and low hydrophilic-lipophilic balances (HLB) allows for the creation of an antiperspirant and/or deodorant composition with desired stability, efficacy, and aesthetic characteristics when combined with the antiperspirant and/or deodorant actives, the carriers, and the preservatives of the present disclosure.

In one embodiment, the antiperspirant and/or deodorant composition includes one or more low HLB surfactants with a low HLB value and one or more high HLB surfactants with a high HLB value.

In some embodiment, the one or more low HLB surfactants have an HLB value from about 1 to about 8. For example, the one or more low HLB surfactants have an HLB value from about 3 to about 6. In one embodiment, the one or more low HLB surfactants have an HLB value of about 5. For example, in some embodiments, the one or more low HLB surfactants include at least one of Steareth-2, Steareth-4, Steareth-6, Steareth-7, Ceteareth-2, Ceteareth-3, Ceteareth-4, and Ceteareth-5. In other embodiments, the one or more low HLB surfactants consist of only one of Steareth-2, Steareth-4, Steareth-6, Steareth-7, Ceteareth-2, Ceteareth-3, Ceteareth-4, or Ceteareth-5 or consists essentially of only one of Steareth-2, Steareth-4, Steareth-6, Steareth-7, Ceteareth-2, Ceteareth-3, Ceteareth-4, or Ceteareth-5. In one example of such an embodiment, the composition contains one low HLB surfactant, where the low HLB surfactant consists of at least 99% of only one of Steareth-2, Steareth-4, Steareth-6, Steareth-7, Ceteareth-2, Ceteareth-3, Ceteareth-4, or Ceteareth-5. In one embodiment, the low HLB surfactant consists essentially of Steareth-2.

In some embodiments, the one or more high HLB surfactants have an HLB value from about 10 to about 18. For example, the one or more high HLB surfactants have an HLB value from about 12 to about 16. In one embodiment, the one or more high HLB surfactants have an HLB value of about 15. For example, in some embodiments, the one or more high HLB surfactants include at least one of Steareth-21, Steareth-10, Steareth-11, Steareth-13, Steareth-15, Steareth-20, Ceteareth-6, Ceteareth-7, Ceteareth-8, Ceteareth-9, Ceteareth-10, Ceteareth-11, and Ceteareth-12.

In other embodiments, the one or more low HLB surfactants consists of only one of Steareth-21, Steareth-10, Steareth-11, Steareth-13, Steareth-15, Steareth-20, Ceteareth-6, Ceteareth-7, Ceteareth-8, Ceteareth-9, Ceteareth-10, Ceteareth-11, or Ceteareth-12, or consist essentially of only one of Steareth-21, Steareth-10, Steareth-11, Steareth-13, Steareth-15, Steareth-20, Ceteareth-6, Ceteareth-7, Ceteareth-8, Ceteareth-9, Ceteareth-10, Ceteareth-11, or Ceteareth-12. In one example of such an embodiment, the composition contains one high HLB surfactant, where the high HLB surfactant consists of at least 99% of only one of Steareth-21, Steareth-10, Steareth-11, Steareth-13, Steareth-15, Steareth-20, Ceteareth-6, Ceteareth-7, Ceteareth-8, Ceteareth-9, Ceteareth-10, Ceteareth-11, and Ceteareth-12. In one embodiment, the high HLB surfactant consists essentially of Steareth-21.

In some embodiment, the one or more low HLB surfactants have an HLB value from about 3 to about 6 and the one or more high HLB surfactants have an HLB value from about 12 to about 16. For example, the one or more low HLB surfactants have an HLB value of about 5 and the one or more high HLB surfactants have an HLB value of about 15. For example, in some embodiments, the one or more surfactants include Steareth-2 and Steareth-21. In other embodiments, the one or more surfactants consist essentially of Steareth-2 and Steareth-21.

In some embodiments, the antiperspirant and/or deodorant composition includes from about 1 % to about 7 % surfactants, based on a total weight of the antiperspirant and/or deodorant composition. For example, the antiperspirant and/or deodorant composition includes from about 3 weight % to about 6 weight % surfactants.

Alternatively, in other embodiments, the antiperspirant and/or deodorant composition may include from about 5 weight % to about 3.5 weight % low HLB surfactants and from about 2.5 weight % to about 1.5 weight % high HLB surfactants. In one embodiment, the antiperspirant and/or deodorant composition includes from about 5 weight % to about 2.5 weight % low HLB surfactants and from about 3.5 weight % to about 1.5 weight % high HLB surfactants. For example, the antiperspirant and/or deodorant composition includes from about 5 weight % to about 3 weight % Steareth-2 and from about 2.5 weight % to about 1 weight % Steareth-21.

In one embodiment, the antiperspirant and/or deodorant composition includes one or more preservatives. In some embodiments, the preservatives improve an antimicrobial characteristic of the antiperspirant and/or deodorant composition to improve storage life or prevent microbial contamination.

In other embodiment, the preservatives may also enhance the functional characteristics of the antiperspirant and/or deodorant composition. For example, in some embodiments, the preservative may provide deodorant or emollient effects to the antiperspirant and/or deodorant composition.

For example, in one embodiment, the one or more preservatives include at least one of phenoxyethanol, caprylyl glycol, ethylhexylglycerin, citric acid, benzoic acid, lactic acid, and combinations thereof. In other embodiments, the one or more preservatives consist essentially of only one of phenoxyethanol, caprylyl glycol, ethylhexylglycerin, citric acid, benzoic acid, or lactic acid, with only trace amounts of other materials. In one example of such an embodiment, the composition contains one preservative, where the preservative consists of at least 99% of only one of phenoxyethanol, caprylyl glycol, ethylhexylglycerin, citric acid, benzoic acid, or lactic acid. In one embodiment, the preservative consists essentially of caprylyl glycol.

In one embodiment, the antiperspirant and/or deodorant composition includes an effective amount of preservatives. For example, the antiperspirant and/or deodorant composition may include an amount of preservatives effective to reduce microbial spoilage of the antiperspirant and/or deodorant composition during storage or use.

In other embodiments, the antiperspirant and/or deodorant composition may include from about 0.1 to about 0.5 weight % preservative. For example, from about 0.1 weight % to about 0.5 weight % caprylyl glycol, from about 0.2 weight % to about 0.4 weight % caprylyl glycol, or about 0.3 weight % caprylyl glycol.

In some embodiments, the antiperspirant and/or deodorant composition may optionally include one or more fragrances. A variety of fragrances can be used in the antiperspirant and/or deodorant compositions if a scented product is desired. For example, in some embodiments, any fragrance suitable for personal care use may be incorporated into the antiperspirant and/or deodorant composition as a non-essential ingredient.

In some embodiments, the antiperspirant and/or deodorant composition may optionally include one or more emollients. For example, the antiperspirant and/or deodorant composition may include non-volatile emollients in any desired amount to achieve a desired emollient effect. Emollients are known in the art and are used to impart a soothing effect on the skin. Classes of non-volatile emollients include non-silicone and silicone emollients. Non-volatile, non-silicone emollients include C12-15 alkyl benzoate. Non-volatile silicone material can be a polyethersiloxane, polyalkyarylsiloxane or polyethersiloxane copolymer. An illustrative non-volatile silicone material is phenyl trimethicone. Non-limiting examples of emollients can be found in U.S. Patent No. 6,007,799. Examples include, but are not limited to, PPG- 14 butyl ether, PPG-15 stearyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl)adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, alkyl benzoate, hydrocyethyl stearate amide, and hydrogenated polyisobutene.

In one embodiment, the emollient is selected from linear silicones, cyclic silicones, hydrocarbons, polyhydroxy alcohols having more than 3 carbon atoms, liquid or solid polyalkyleneglycol ethers containing a polypropylene glycol (PPG) moiety and terminating in an alkyl ether, and combinations thereof. In another embodiment, the emollient is a nonvolatile silicone, such as dimethiconol or a longer chain dimethicone.

In one embodiment, the antiperspirant and/or deodorant composition includes from about 0.1 % to about 30 % emollients, based on a total weight of the antiperspirant and/or deodorant composition. For example, the antiperspirant and/or deodorant composition may include from about 1 weight % to about 25 weight % emollients or from about 1 weight % to about 15 weight % emollients.

Generally, viscosity is a characteristic that is considered when creating antiperspirant and/or deodorant compositions, and especially roll-on formulations. For example, when the viscosity of a roll-on antiperspirant and/or deodorant composition is too low, it may become too runny and it may leak from the roll-on dispenser during storage or use. In some cases, this will not only affect the delivery and aesthetics of the antiperspirant and/or deodorant composition but may also affect the homogeneity of the ingredients in the antiperspirant and/or deodorant composition. On the other hand, if the viscosity of the antiperspirant and/or deodorant composition is too high, packaging into and delivery through a roll-on dispenser will be difficult.

Accordingly, it is desirable to select ingredients, and their specific amounts, for antiperspirant and/or deodorant compositions that achieve an advantageous range of viscosity to ensure product manufacturability, stability, and quality, as well as adequate delivery through roll-on dispensers.

In some embodiments, the viscosity of the antiperspirant and/or deodorant composition is from about 900 centipoise (cPs) to about 4,000 cPs at 25° C. In other embodiments, the viscosity of the antiperspirant and/or deodorant composition is from about 1,100 cPs to about 3,500 cPs at 25° C. In one embodiment, the viscosity of the antiperspirant and/or deodorant composition is from about 1,500 cPs to about 2,500 cPs at 25° C. Viscosity is measured in centipoise (cPs) by using a Brookfield Viscometer at 25° C for a minute with spindle 4 at an RPM setting of 20.

In embodiments of the present disclosure, the antiperspirant and/or deodorant composition achieves a desirable viscosity using the five to seven types of ingredients described herein and does not require rheology modifiers to achieve a target viscosity. For example, in some embodiments, the antiperspirant and/or deodorant composition does not include thickeners or viscosity modifiers, such as hydroxyethyl cellulose, modified hydroxyethyl cellulose, and scleroglucan.

In other embodiments, the antiperspirant and/or deodorant composition does not include gelling agents, such as stearyl alcohol, dibenzylidene sorbitol, and polyethylene glycol, or other rheology modifiers.

In some embodiments, the antiperspirant and/or deodorant composition displays pearlescence. For examples, in some embodiments, the antiperspirant and/or deodorant composition has a pearl-like iridescent sheen, a glowing appearance, or a luster resembling that of mother of pearl. In other embodiments, the antiperspirant and/or deodorant composition displays a high luster and sheen that provides a pearlescent appearance.

### EXAMPLES

Aspects of the present disclosure may be further understood by referring to the following examples. The examples are illustrative, and are not intended to be limiting embodiments thereof Tables 1-3 illustrate antiperspirant or deodorant compositions according to embodiments of the present disclosure. Example 1 illustrates an embodiment of a process to make the antiperspirant or deodorant compositions of Table 1 and Example 2 illustrates an embodiment of a process to make the antiperspirant or deodorant compositions of Tables 2-3.

**Table 1**

| **Composition 1** | |
|---|---|
| **Ingredient** | **Weight %** |
| Potassium Aluminum Sulphate (deodorant active) | 2% |
| Steareth -2 | 3.5 % |
| Steareth -21 | 2.5 % |
| Caprylyl Glycol | 0.5 % |
| Water | Q.S. |
| **Total** | **100 %** |

**Table 2**

| **Composition 2** | |
|---|---|
| **Ingredient** | **Weight %** |
| Aluminium Chlorohydrate (antiperspirant active) | 8% |
| Steareth -2 | 3.5 % |
| Steareth -21 | 2.5 % |
| Caprylyl Glycol | 0.5 % |
| Water | Q.S. |
| **Total** | **100 %** |

**Table 3**

| **Composition 3** | |
|---|---|
| **Ingredient** | **Weight %** |
| Aluminium Chlorohydrate (antiperspirant active) | 12% |
| Steareth -2 | 3.5% |
| Steareth -21 | 2.5% |
| Caprylyl Glycol | 0.5% |
| Water | Q.S. |
| **Total** | **100 %** |

### Example 1

The antiperspirant and/or deodorant composition of Table 1 was prepared as follows: the formula amounts of the surfactants were mixed together and heated to 80 °C. The formula amounts of water and preservative were mixed together and heated to 80 °C. The formula amounts of deodorant active was added to the mixture of water and preservative, and the resulting mixture was added to the surfactant mixture. The complete mixture was homogenized for 3 minutes and allowed to cool to room temperature.

### Example 2

The antiperspirant and/or deodorant compositions of Tables 2-3 were prepared as follows: the formula amounts of the surfactants were mixed together and heated to 80 °C. The formula amounts of water and preservative were mixed together and heated to 80 °C. The resulting mixture was then added to the surfactant mixture, homogenized for 3 minutes, and allowed to cool to 65 °C. The formula amounts of antiperspirant active was then added to the mixture and allowed to cool to room temperature.

The antiperspirant and/or deodorant compositions of Tables 1-3 were then analyzed for their micro-robustness using the APET (antimicrobial preservation effectiveness test), which measures the ability of a formulation to resist the growth of yeast, mold, and bacteria.

The APET test is a familiar test used in the cosmetic and personal care industry. The APET test is conducted under either aged or unaged conditions. Aged means that the product being tested has been maintained at a temperature of 40 °C and 75% relative humidity for 8 weeks in final packaging, whereas the unaged test is performed on fresh product after manufacture. The test measures the growth of an inoculum of bacteria or mold in the presence of the tested composition. APET results in either a pass or fail rating. A passing APET test requires a greater than 99.9% reduction in the level of bacteria in the inoculum and a greater than 90.0% reduction in the level of mold in the inoculum after 7 days.

As illustrated in Table 4, the antiperspirant and/or deodorant compositions of Tables 1-3 meet APET acceptance criteria.

**Table 4**

| | **Composition 1** | **Composition 2** | **Composition 3** |
|---|---|---|---|
| APET | Sample meet APET | Sample meet APET | Sample meet APET |
| | acceptance criteria | acceptance criteria | acceptance criteria |
| | Bacteria: > 99.9% | Bacteria: > 99.9% | Bacteria: > 99.9% |
| | reduction | reduction | reduction |
| | Mold :> 90.0% | Mold :> 90.0% | Mold :> 90.0% |
| | reduction | reduction | reduction |

The antiperspirant and/or deodorant compositions of Tables 1-3 were then also analyzed for stability in terms of odor, color, and appearance before and after aging, after exposure to sunlight, and after repeated freeze/thaw cycles. In particular, samples of the antiperspirant and/or deodorant compositions were aged for up to 13 weeks, which is the extent of time commonly used to predict a 24 month shelf life under normal conditions. The compositions of Tables 1-3 were stored at different temperatures and relative humidity levels for up to 13 weeks. In addition, the compositions of Tables 1 -3 were exposed to natural sunlight for 4 weeks and subjected to repeated freeze/thaw cycles for 7 days.

Odor, color, and appearance was evaluated by a trained stability evaluator comparing a reference sample stored at cold temperature for each composition to samples of the respective compositions at different time and temperature points, after prolonged exposure to sunlight, and after 7 days of freeze/thaw cycles. Odor, color, and appearance was rated as acceptable based on the odor, color, and appearance of the reference sample.

As illustrated in Tables 5-7 below, the compositions of Tables 1-3 displayed no spoilage or significant degradation in color, odor, or appearance.

**Table 5**

| **Stability Data - Composition 1** | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

**Table 6**

| **Stability Data - Composition 2** | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

**Table 7**

| **Stability Data - Composition 3** | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

Mildness and irritation of the antiperspirant and/or deodorant compositions of the present disclosure, such as those of Tables 1-3 was also determined to be comparable to commercial counterparts. In particular, measurements of Interleukin 1 alpha (IL-1α) protein were used to determine skin irritation potential of the composition of Table 1. IL-1α is continuously released by skin cells. However, a higher level of IL-1α release may be used as an indicator of higher irritation potential for a substance contacting the skin. Accordingly, *in vitro* experiments to evaluate skin irritation were performed using human skin models (SIT 200 skin irritation model, MatTek Corporation, Ashland, MA) and IL-1α ELISA assay kits (R&D Systems, Minneapolis, MN 55413). In particular, SIT 200 skin model samples were treated topically with 30 ul of Composition 1 for 1 hour in a tissue culture incubator. An untreated control was also incubated to establish a comparison basis. Following incubation, the skin samples were washed with phosphate buffer saline (PBS), placed in a culture medium, and incubated for 24 hours. The cell culture media was then collected for evaluation using the IL-1α release assay, the results of which are illustrated in Table 8 below.

**Table 8**

| **Irritability Data** | | |
|---|---|---|
| | **Untreated Control** | **Composition 1** |
| IL-1α Concentration | 21 pg / mL | 19 pg / mL |

As illustrated in Table 8, the antiperspirant and/or deodorant compositions of the present disclosure, such as those of Table 1, had a lower level of IL-1α release when compared to an untreated control, indicating a low potential for skin irritability.

The compositions of Tables 1-3 were loaded into roll-on dispensers and applied to surfaces to measure viscosity and delivery. The compositions of Tables 1-3 had a comparable delivery through roll-on dispensers as commercial counterparts, each composition having a viscosity between 1000-4000 cps.

In some embodiments, the antiperspirant and/or deodorant compositions of the present disclosure have a pearlescent appearance as evaluated by a panel of people. In particular, as illustrated in Table 9, a panel of 9 people was used to evaluate the pearlescent appearance of antiperspirant and/or deodorant compositions of the present disclosure using a combination of high HLB and low HLB surfactants as the pearling agent. The compositions of Table 9 are similar to those of Tables 1-3 but used different amounts of Steareth-2 and Steareth-21 as the pearling agent.

**Table 9**

| | | | Pearlescent Appearance? | | |
|---|---|---|---|---|---|
| | Steareth-2 | Steareth-21 | Yes | Maybe | No |
| Composition 4 | 25% | 75 % | 9 | - | - |
| Composition 5 | 0% | 100% | - | 1 | 8 |
| Composition 6 | 66.6 % | 33.3 % | 9 | - | - |
| Composition 7 | 50% | 50% | 7 | - | -2 |
| Composition 8 | 33.3 % | 66.6 % | 7 | - | 2 |
| Composition 9 | 75 % | 25% | 8 | - | 1 |

As illustrated in Table 9, when including combinations of high HLB and low HLB surfactants per embodiments of the present disclosure, visual evaluation of the deodorant and/or antiperspirant compositions of Table 9 by a panel of people indicated they possessed a recognizable pearlescent appearance.

The present disclosure has been described with reference to exemplary embodiments. Although a limited number of embodiments have been shown and described, it will be appreciated by those skilled in the art that changes may be made in these embodiments without departing from the principles and spirit of preceding detailed description. It is intended that the present disclosure be construed as including all such modifications and alterations insofar as they come within the scope of the appended claims or the equivalents thereof.

Further aspects of this application are:
1. An antiperspirant and/or deodorant composition, comprising:
   between 60 weight % and 95 weight % carrier, based on a total weight of the antiperspirant and/or deodorant composition;
   between 2 weight % and 25 weight % antiperspirant and/or deodorant active, based on the total weight of the antiperspirant and/or deodorant composition;
   between 1 weight % and 7 weight % surfactant, based on the total weight of the antiperspirant and/or deodorant composition; and
   between 0.1 weight % and 0.5 weight % preservative, based on the total weight of the antiperspirant and/or deodorant composition.
2. The antiperspirant and/or deodorant composition of aspect 1, wherein the antiperspirant and/or deodorant composition does not comprise rheology modifiers, viscosity modifiers, or thickening agents.
3. The antiperspirant and/or deodorant composition of aspect 1, wherein the antiperspirant and/or deodorant composition does not comprise gelling agents.
4. The antiperspirant and/or deodorant composition of aspect 1, wherein a viscosity of the antiperspirant and/or deodorant composition is from about 900 to about 4000cps.
5. The antiperspirant and/or deodorant composition of aspect 1, wherein the antiperspirant and/or deodorant composition exhibits pearlescense.
6. The antiperspirant and/or deodorant composition of aspect 1, wherein the carrier consists essentially of water.
7. The antiperspirant and/or deodorant composition of aspect 1, wherein the antiperspirant and/or deodorant active consists essentially of an antiperspirant active.
8. The antiperspirant and/or deodorant composition of claim 1, wherein the antiperspirant active consists essentially of aluminum chlorohydrate.
9. The antiperspirant and/or deodorant composition of aspect 1, wherein the antiperspirant and/or deodorant active consists essentially of a deodorant active.
10. The antiperspirant and/or deodorant composition of aspect 1, wherein the deodorant active consists essentially of potassium aluminum sulfate.
11. The antiperspirant and/or deodorant composition of aspect 1, wherein the surfactant comprises one or more low HLB surfactants and one or more high HLB surfactants, and
   wherein the one or more low HLB surfactants have an HLB value from about 3 to about 6 and the one or more high HLB surfactants have an HLB value from about 12 to about 16.
12. The antiperspirant and/or deodorant composition of aspect 11, wherein the surfactant consists essentially of Steareth-2 and Steareth-21.
13. The antiperspirant and/or deodorant composition of aspect 1, wherein the preservative consists essentially of caprylyl glycol.
14. The antiperspirant and/or deodorant composition of aspect 1, further comprising at least one of a fragrance, and an emollient.
15. The antiperspirant and/or deodorant composition of aspect 1, consisting essentially of:
   between 60 weight % and 90 weight % water as the carrier;
   between 8 weight % and 12 weight % aluminum chlorohydrate as the deodorant and/or antiperspirant active;
   between 1 weight % and 6 weight % Steareth-2 and Steareth-21 as the surfactant;
   between 0.1 weight % and 0.5 weight % caprylyl glycol as the preservative; and
   between 0 weight % and 30 weight % emollient.
16. The antiperspirant and/or deodorant composition of aspect 1, consisting essentially of:
   between 60 weight % and 95 weight % water;
   between 2 weight % and 5 weight % potassium aluminum sulfate;
   between 1 weight % and 6 weight % Steareth-2 and Steareth-21;
   an effective amount of preservative; and
   between 0 weight % and 15 weight % emollient.
17. An antiperspirant and/or deodorant composition, comprising:
   between 60 weight % and 95 weight % carrier, based on a total weight of the antiperspirant and/or deodorant composition;
   between 2 weight % and 25 weight % antiperspirant and/or deodorant active, based on the total weight of the antiperspirant and/or deodorant composition;
   between 1 weight % and 6 weight % surfactant, based on the total weight of the antiperspirant and/or deodorant composition;
   an effective amount of a preservative; and
   between 0 weight % and 15 weight % emollient, based on the total weight of the antiperspirant and/or deodorant composition,
   wherein the antiperspirant and/or deodorant composition does not comprise rheology modifiers, viscosity modifiers, thickening agents, or gelling agents,
   wherein a viscosity of the antiperspirant and/or deodorant composition is from about 900 to about 4000cps, and
   wherein the antiperspirant and/or deodorant composition exhibits pearlescense.
18. The antiperspirant and/or deodorant composition of aspect 17:
   wherein the carrier consists essentially of water,
   wherein the antiperspirant and/or deodorant active consists essentially of at least one of aluminum chlorohydrate and potassium aluminum sulfate,
   wherein the surfactant comprises one or more low HLB surfactants and one or more high HLB surfactants, and wherein the one or more low HLB surfactants have an HLB value from about 3 to about 6 and the one or more high HLB surfactants have an HLB value from about 12 to about 16, and
   wherein the preservative consists essentially of caprylyl glycol.
19. A pearlescent antiperspirant and/or deodorant composition, comprising:
   between 2 weight % and 25 weight % antiperspirant and/or deodorant active, based on the total weight of the antiperspirant and/or deodorant composition; and
   between 2.5 weight % and 3.5 weight % surfactant, based on the total weight of the antiperspirant and/or deodorant composition,
   wherein the surfactant comprises one or more low HLB surfactants and one or more high HLB surfactants, and wherein the one or more low HLB surfactants have an HLB value from about 3 to about 6 and the one or more high HLB surfactants have an HLB value from about 12 to about 16,
   wherein the antiperspirant and/or deodorant composition does not comprise rheology modifiers, viscosity modifiers, thickening agents, or gelling agents,
   wherein a viscosity of the antiperspirant and/or deodorant composition is from about 900 to about 4000cps.
20. The pearlescent antiperspirant and/or deodorant composition of aspect 19:
   wherein the antiperspirant and/or deodorant active consists essentially of at least one of aluminum chlorohydrate and potassium aluminum sulfate, and
   wherein the surfactant comprises Steareth-2 and Steareth-21.

## Claims

1. An antiperspirant and/or deodorant composition, comprising:
between 60 weight % and 95 weight % carrier, based on a total weight of the antiperspirant and/or deodorant composition;
between 2 weight % and 25 weight % antiperspirant and/or deodorant active, based on the total weight of the antiperspirant and/or deodorant composition;
between 1 weight % and 7 weight % surfactant, based on the total weight of the antiperspirant and/or deodorant composition; and
an effective amount of a preservative.

2. The antiperspirant and/or deodorant composition of claim 1, wherein the antiperspirant and/or deodorant composition does not comprise rheology modifiers, viscosity modifiers, or thickening agents.

3. The antiperspirant and/or deodorant composition of claim 1, wherein the antiperspirant and/or deodorant composition does not comprise gelling agents.

4. The antiperspirant and/or deodorant composition of claim 1, wherein a viscosity of the antiperspirant and/or deodorant composition is from about 900 to about 4000cps.

5. The antiperspirant and/or deodorant composition of claim 1, wherein the carrier consists essentially of water.

6. The antiperspirant and/or deodorant composition of claim 1, wherein the antiperspirant and/or deodorant active consists essentially of an antiperspirant active, and optionally wherein the antiperspirant active consists essentially of aluminum chlorohydrate; or wherein the antiperspirant and/or deodorant active consists essentially of a deodorant active, and optionally wherein the deodorant active consists essentially of potassium aluminum sulfate.

7. The antiperspirant and/or deodorant composition of claim 1, wherein the surfactant comprises one or more low HLB surfactants and one or more high HLB surfactants, and
wherein the one or more low HLB surfactants have an HLB value from about 3 to about 6 and the one or more high HLB surfactants have an HLB value from about 12 to about 16.

8. The antiperspirant and/or deodorant composition of claim 7, wherein the surfactant consists essentially of Steareth-2 and Steareth-21.

9. The antiperspirant and/or deodorant composition of claim 1, consisting essentially of:
between 60 weight % and 90 weight % water as the carrier;
between 2 weight % and 5 weight % potassium aluminum sulfate as the deodorant and/or antiperspirant active;
between 1 weight % and 6 weight % Steareth-2 and Steareth-21 as the surfactant;
an effective amount of a preservative ; and
between 0 weight % and 30 weight % emollient.

10. The antiperspirant and/or deodorant composition of claim 1, consisting essentially of:
between 60 weight % and 95 weight % water;
between 2 weight % and 5 weight % potassium aluminum sulfate;
between 1 weight % and 6 weight % Steareth-2 and Steareth-21;
an effective amount of preservative; and
between 0 weight % and 15 weight % emollient.

11. An antiperspirant and/or deodorant composition, comprising:
between 60 weight % and 95 weight % carrier, based on a total weight of the antiperspirant and/or deodorant composition;
between 2 weight % and 25 weight % antiperspirant and/or deodorant active, based on the total weight of the antiperspirant and/or deodorant composition;
between 1 weight % and 6 weight % surfactant, based on the total weight of the antiperspirant and/or deodorant composition;
an effective amount of a preservative; and
between 0 weight % and 15 weight % emollient, based on the total weight of the antiperspirant and/or deodorant composition,
wherein the antiperspirant and/or deodorant composition does not comprise rheology modifiers, viscosity modifiers, thickening agents, or gelling agents,
wherein a viscosity of the antiperspirant and/or deodorant composition is from about 900 to about 4000cps, and
wherein the antiperspirant and/or deodorant composition exhibits pearlescense.

12. The antiperspirant and/or deodorant composition of claim 11:
wherein the carrier consists essentially of water,
wherein the antiperspirant and/or deodorant active consists essentially of at least one of aluminum chlorohydrate and potassium aluminum sulfate, and
wherein the surfactant comprises one or more low HLB surfactants and one or more high HLB surfactants, and wherein the one or more low HLB surfactants have an HLB value from about 3 to about 6 and the one or more high HLB surfactants have an HLB value from about 12 to about 16, and
wherein the preservative consists essentially of caprylyl glycol and sodium benzoate.

13. A pearlescent antiperspirant and/or deodorant composition, comprising:
between 2 weight % and 25 weight % antiperspirant and/or deodorant active, based on the total weight of the antiperspirant and/or deodorant composition; and
between 2.5 weight % and 6 weight % surfactant, based on the total weight of the antiperspirant and/or deodorant composition,
wherein the surfactant comprises one or more low HLB surfactants and one or more high HLB surfactants, and wherein the one or more low HLB surfactants have an HLB value from about 3 to about 6 and the one or more high HLB surfactants have an HLB value from about 12 to about 16,
wherein the antiperspirant and/or deodorant composition does not comprise rheology modifiers, viscosity modifiers, thickening agents, or gelling agents,
wherein a viscosity of the antiperspirant and/or deodorant composition is from about 900 to about 4000cps.

14. The pearlescent antiperspirant and/or deodorant composition of claim 13:
wherein the antiperspirant and/or deodorant active consists essentially of at least one of aluminum chlorohydrate and potassium aluminum sulfate, and
wherein the surfactant comprises Steareth-2 and Steareth-21.

15. An antiperspirant and/or deodorant composition used for reducing IL-1α release and skin irritability, comprising:
between 60 weight % and 95 weight % carrier, based on a total weight of the antiperspirant and/or deodorant composition;
between 2 weight % and 25 weight % antiperspirant and/or deodorant active, based on the total weight of the antiperspirant and/or deodorant composition, wherein the deodorant active consists essentially of potassium aluminum sulfate;
between 1 weight % and 7 weight % surfactant, based on the total weight of the antiperspirant and/or deodorant composition; and
an effective amount of a preservative.
